(19) Europäisches Patentamt
European Patent Office
Office européen des brevets

(11) **EP 2 346 393 B1**

(12) **EUROPÄISCHE PATENTSCHRIFT**

(45) Veröffentlichungstag und Bekanntmachung des Hinweises auf die Patenterteilung:
**23.12.2015   Patentblatt 2015/52**

(21) Anmeldenummer: **09748040.4**

(22) Anmeldetag: **29.10.2009**

(51) Int Cl.:
***A61B 5/0215*** *(2006.01)*

(86) Internationale Anmeldenummer:
**PCT/EP2009/007729**

(87) Internationale Veröffentlichungsnummer:
**WO 2010/057565 (27.05.2010 Gazette 2010/21)**

(54) **VERFAHREN ZUR DURCHFÜHRUNG EINES NULLPUNKTABGLEICHS EINES PATIENTENÜBERWACHUNGSSYSTEMS UND DAZUGEHÖRIGES PATIENTENÜBERWACHUNGSSYSTEM**

METHOD FOR CARRYING OUT A ZERO POINT COMPARISON IN A PATIENT MONITORING SYSTEM AND CORRESPONDING PATIENT MONITORING SYSTEM

PROCÉDÉ DE RÉGLAGE AU POINT ZÉRO D'UN SYSTÈME DE SURVEILLANCE DE PATIENTS ET SYSTÈME DE SURVEILLANCE DE PATIENTS ASSOCIÉ

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **18.11.2008   DE 102008058101**

(43) Veröffentlichungstag der Anmeldung:
**27.07.2011   Patentblatt 2011/30**

(73) Patentinhaber: **Hyb D.O.O.**
**8310 Sentjernej (SI)**

(72) Erfinder:
• **BECK, Bernd**
**72414 Rangendingen (DE)**
• **PAVLIN, Marko**
**8000 Novo Mesto (SI)**

(74) Vertreter: **DREISS Patentanwälte PartG mbB**
**Friedrichstrasse 6**
**70174 Stuttgart (DE)**

(56) Entgegenhaltungen:
EP-A1- 1 574 164     WO-A2-2007/058826
DE-A1- 10 009 591    DE-U1- 9 408 557
US-A- 5 566 676      US-B1- 6 290 652

**Beschreibung**

Die Erfindung betrifft ein Verfahren zur Durchführung eines Nullpunktabgleichs eines Patientenüberwachungssystems mit einer am Patienten abgenommene Messdaten über eine Funkstrecke versendenden Sendeeinheit und mit wenigstens einer Empfangseinheit zum Empfangen der von der Sendeeinheit ausgesendeten Messdaten, wobei die am Patienten abgenommenen Messdaten Drucksignale sind, die über eine flüssigkeitsgefüllte Druckmessleitung einem die Drucksignale in elektrische Signale umwandelnden und an die Sendeeinheit weiterleitenden Druckmesswandler zugeführt werden, und wobei die von der Empfangseinheit empfangenen Messdaten insbesondere an einem Monitor angezeigt werden.

Derartige Patientenüberwachungssysteme sind beispielsweise aus der EP 1 574 164 A1 vorbekannt. Problematisch hat sich in der Praxis die Durchführung eines Nullpunktabgleichs des Patientenüberwachungssystems herausgestellt. Ein Nullpunktabgleich ist erforderlich, da jeder Druckmesswandler eine unterschiedliche, sogenannte Offset-Spannung $V_{ofs}$ aufweist, welche den Absolutwert des Messergebnisses verfälscht. Insofern ist es erforderlich, nach der Herstellung der Verbindung zwischen dem Druckmesswandler und dem Monitor die Nulllinie des Monitors derart einzustellen, dass ein dem Umgebungsdruck entsprechendes Eingangssignal des Monitors auf einer Nulllinie des Monitors liegt. Die US 5,566,676 A offenbart ein Datenerfassungsgerät, wobei das Datenerfassungsgerät eine aufgrund eines zu messenden Drucks erzeugte Spannung an ein Display sendet und wobei am Datenerfassungsgerät ein Null-Druck-Schalter vorgesehen ist. Beim Drücken dieses Schalters wird das Display genullt.

Mit der vorliegenden Erfindung soll die Durchführung eines derartigen Nullpunktabgleichs vereinfacht werden, wobei ein einfaches Verbinden der Empfangseinheiten mit wechselnden Monitoren ermöglicht werden soll.

Diese Aufgabe wird durch ein Verfahren mit den Merkmalen des Anspruchs 1 gelöst. Das Verfahren zeichnet sich folglich dadurch aus, dass im Normalbetrieb die von der Sendeeinheit versendeten Messdaten um den Wert $V_{ofs}$ bereinigt an die Empfangseinheit versendet werden.

Die Durchführung des Verfahrens hat den Vorteil, dass die von der Sendeeinheit versendeten Messdaten bereits um die Offset-Spannung $V_{ofs}$ bereinigt an die Empfangseinheit versendet werden. Die Berücksichtigung der Offset-Spannung $V_{ofs}$ erfolgt also nicht auf Seiten des Empfängers oder des Monitors, sondern erfolgt sendeseitig. Da das von der Sendeeinheit an die Empfangseinheit versendete Signal aufgrund der Bereinigung der Offset-Spannung des Messwandlers korrekte Absolutwerte aufweist, können diese als solche von der Empfangseinheit empfangen und am Monitor graphisch dargestellt werden. Gegebenenfalls ist es lediglich erforderlich, einmalig einen Abgleich zwischen der Empfangseinheit und dem Monitor durchzuführen.

Bei der Erfindung ist es ferner vorteilhaft, wenn mit der Empfangseinheit ein Basissignal zum Abgleichen der am Monitor angezeigten Signale generierbar ist, aufgrund dessen die Nulllinie des Monitors auf einen Basiswert einstellbar ist. Vorteilhafterweise entspricht das von der Empfangseinheit generierte Basissignal einem Signal der Sendeeinheit, das aus dem Umgebungsluftdruck $p_0$ resultiert, wobei dann der Basiswert einem Nullwert entspricht. In diesem Fall wird folglich aufgrund des empfangseinheitenseitig generierten Basissignals die Nulllinie des Monitors auf den Nullwert gesetzt. Gemäß der Erfindung ist es allerdings nicht zwangsweise erforderlich, dass das Basissignal einem Nullwertsignal entspricht; das Basissignal kann beispielsweise auch einem X-Wert entsprechen und die Nulllinie des Monitors kann dann in Abhängigkeit des X-Wert-Signals entsprechend verstellt werden.

Insbesondere ist denkbar, dass aufgrund des Basissignals der Empfangseinheit die Nulllinie des Monitors händisch auf den entsprechenden Wert eingestellt wird. Hierfür kann monitorseitig beispielsweise ein entsprechender Drehregler oder eine entsprechende Taste vorgesehen sein.

Bei einer weiteren vorteilhaften Ausgestaltung der Erfindung ist vorgesehen, dass die Nulllinie des Monitors aufgrund eines von der Empfangseinheit generierten Nullpunktabgleichssignals automatisch eingestellt wird. Folglich wird beim Nullpunktabgleich in der Empfangseinheit ein Nullpunktabgleichssignal generiert, welches vom Monitor erkannt wird. Aufgrund dieses Nullpunktabgleichssignals wird dann, über eine entsprechende monitorseitige Einheit die Nulllinie des Monitors selbsttätig auf den Wert Null verschoben.

Dabei ist denkbar, dass während oder nach der Bestimmung und insbesondere auch der Speicherung der Offset-Spannung $V_{ofs}$ in der Sendeeinheit ein Nullpunktabgleichsbefehl in die Empfangseinheit versendet wird, aufgrund dessen das Nullpunktabgleichssignal von der Empfangseinheit generiert und die Nulllinie des Monitors entsprechend händisch einstellbar ist und/oder automatisch eingestellt wird.

Vorteilhafterweise wird das Verfahren voll automatisch durchgeführt, das heißt, dass während der Bestimmung bzw. Speicherung der Offset-Spannung $V_{ofs}$ die Nulllinie des Monitors automatisch aufgrund des Nullpunktabgleichsbefehls und des Nullpunktabgleichssignals eingestellt wird.

Die eingangs genannte Aufgabe wird auch gelöst durch ein erfindungsgemäßes Patientenüberwachungssystem. Ein derartiges Patientenüberwachungssystem sieht neben einer Sendeeinheit und einer Empfangseinheit in oder an der Druckmessleitung ein Ventil vor, welches in einer Betriebsstellung eine Verbindung zwischen dem Patienten und dem Druckmesswandler herstellt und welches zur Durchführung des Nullpunktabgleichs in einer Abgleichstellung eine Verbindung zwischen einer am Umgebungsluftdruck anstehenden Flüssigkeit und dem Druckmesswandler herstellt. In dieser Nullpunktabgleichstellung des Ventils kann folglich der Nullpunktabgleich gemäß dem erfindungsgemäßen Verfahren durchgeführt werden. Die Sendeeinheit umfasst dabei Auswertmittel, welche die in der Abgleichsstellung des Ventils aus dem Umgebungsluftdruck $p_0$ resultierende, vom Druckmesswandler erzeugte Offset-Spannung $V_{ofs}$ bestimmen und welche die von der Sendeeinheit an die Empfangseinheit versendeten Messdaten um den Wert der Offset-Spannung $V_{ofs}$ bereinigen.

Die Sendeeinheit kann dabei insbesondere eine Speicherfunktion aufweisen, die zur Speicherung der Offset-Spannung $V_{ofs}$ betätigbar ist. Nachdem folglich das Ventil in die Abgleichsstellung gestellt wurde, wird zur Durchführung des Nullpunktabgleichs die Speicherfunktion, beispielsweise durch Drücken einer senderseitigen Speichertaste, ausgeführt. Denkbar ist auch, dass nach dem Umstellen des Ventils innerhalb eines gewissen Zeitraums die Offset-Spannung $V_{ofs}$ automatisch gespeichert wird.

Ferner ist vorteilhaft, wenn die Empfangseinheit eine Abgleichfunktion aufweist, die zur Generierung des Basissignals betätigbar ist. Nach der Betätigung der Abgleichtaste kann innerhalb eines vorgebbaren Zeitraums die Nulllinie des Monitors auf den tatsächlichen Nullwert eingestellt werden. Das Einstellen kann dabei, wie bereits erwähnt, entweder händisch oder automatisch erfolgen.

Erfindungsgemäß ist ferner denkbar, dass die Sendeeinheit eine Abgleichfunktion und die Empfangseinheit Mittel zur Generierung eines Nullpunktabgleichsbefehls derart aufweist, dass durch Betätigung der Abgleichfunktion von der Sendeeinheit ein Nullpunktabgleichsbefehls an die Empfangseinheit übersendet wird und aufgrund des Nullpunktabgleichsbefehls in der Empfangseinheit ein Nullpunktabgleichssignal generiert wird. Die Abgleichfunktion kann beispielsweise durch Betätigen einer entsprechenden Taste an der Sendeeinheit und/oder der Empfangseinheit ausgeführt werden.

Gemäß der Erfindung kann zudem vorgesehen sein, dass die Empfangseinheit Mittel zur automatischen Einstellung der Nulllage des Monitors derart aufweist, dass die Nulllinie des Monitors aufgrund des Nullpunktabgleichssignals automatisch eingestellt wird.

Eine weitere vorteilhafte Ausführungsform der Erfindung sieht vor, wenn der Monitor in die Empfangseinheit integriert ist. Hierdurch wird eine Empfangseinheit bereitgestellt, die kompakt und insbesondere auch mobil ausgeführt sein kann.

Bei einer anderen Ausführungsform der Erfindung ist denkbar, dass der Monitor separat von der Empfangseinheit ausgebildet ist und einen Anschluss aufweist, über den das von der Empfangseinheit generierte Nullpunktabgleichssignal dem Monitor zuführbar ist. Aufgrund des entsprechenden Anschlusses bzw. einer entsprechenden Schnittstelle kann folglich das Nullpunktabgleichssignal dem Monitor zugeführt werden. Aufgrund des Nullpunktabgleichssignals kann, wie bereits beschrieben, die Nulllinie des Monitors automatisch eingestellt werden.

Weitere Einzelheiten und vorteilhafte Ausgestaltungen der Erfindung sind der nachfolgenden Beschreibung zu entnehmen, anhand derer die Erfindung beschrieben und erläutert wird.

Es zeigen:

Figur 1      eine schematische Darstellung eines Nullpunktabgleichs bei vorbekannten Patientenüberwachungssystemen;

Figur 2      eine schematische Darstellung einer Durchführung eines Nullpunktabgleichsystems bei einem erfindungsgemäßen Patientenüberwachungssystem; und

Figur 3      das Patientenüberwachungssystem gemäß Fig. 3 im Betrieb,

Figur 4      die Durchführung des Nullpunktabgleichs bei einem Patientenüberwachungssystem mit mehreren Empfängern,

Figur 5      Diagramm Zusammenhang zwischen dem Drucksignal p und der Ausgangsspannung $V_{out}$ und

Figur 6      Diagramm der um den Wert $V_{ofs}$ bereinigten Ausgangsspannung $V_{out}$

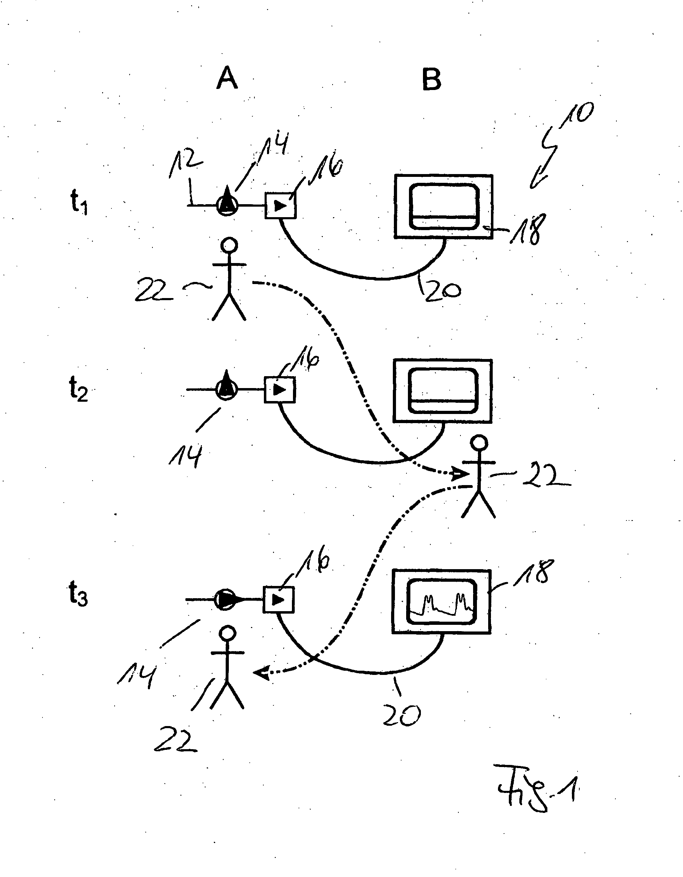

In der Figur 1 ist ein bekanntes Patientenüberwachungssystem 10 dargestellt, das eine flüssigkeitsgefüllte Druckmessleitung 12, ein Ventil 14, einen Druckmesswandler 16 und einen Monitor 18 umfasst. Der Druckmesswandler 16 wandelt dabei die analogen Drucksignale in elektrische Signale um und leitet diese über eine Kabelverbindung 20 dem Monitor zu. Bei den am Patienten abgenommenen Drucksignalen kann es sich insbesondere um intravenöse Blutdrucksignale handeln.

Da jeder Druckmesswandler beaubedingt eine unterschiedliche Offset-Spannung $V_{ofs}$ aufweist, welche in der Regel bei einem Null bar Referenzdruck nicht gleich Null Volt ist, ist es erforderlich, das am Monitor 18 angezeigte Messsignal um diese Spannung $V_{ofs}$ zu bereinigen. Erst ergeben sich am Monitor 18 korrekt angezeigte Absolutwerte.

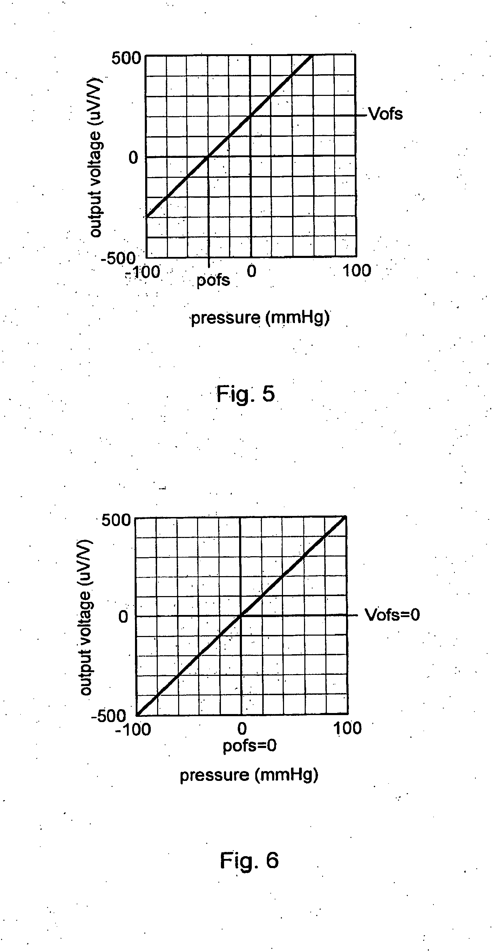

In Figur 5 ist ein typischer Zusammenhang zwischen dem an dem Druckmesswandler 16 gemessenen Drucksignal p und der zugehörigen Ausgangsspannung $V_{out}$ des Druckmesswandlers dargestellt.

Deutlich zu erkennen ist, dass bei einem Druck p gleich Null eine Ausgangsspannung $V_{out}$ vorhanden ist, welche ungleich Null ist. Diese von dem Wert Null abweichende Offset-Spannung $V_{ofs}$ verfälscht das am Monitor 18 angezeigte Signal.

Um eine um den Wert $V_{ofs}$ bereinigte Ausgangsspannung $V_{out}$ zu erhalten, ist die Ausgangsspannung $V_{out}$ um die Offset-Spannung $V_{ofs}$ zu reduzieren. Eine um den Wert $V_{ofs}$ bereinigte Ausgangsspannung $V_{out}$ bei einem Druck p gleich Null ist Figur 6 zu entnehmen.

Die Ausgangsspannung $V_{out}$ des Druckmesswandlers 16 lässt sich darstellen als:

$$V_{out} = (V_{B}s)(p + p_{ofs}) \qquad (1).$$

Dabei ist $V_B$ die Brückenerregerspannung, s die Sensitivität des Druckmesswandlers und $p_{ofs}$ der Offset-Druck. Bei einem Wert p gleich Null ergibt sich also die Offset-Spannung $V_{ofs}$:

$$V_{ofs} = (V_{B}s)p_{ofs} \qquad (2)$$

Die Sensitivität s entspricht dabei beispielsweise

$$s = 5\mu V /V /mmHg \qquad (3).$$

Am Monitor 18 muss die Ausgangsspannung $V_{out}$ um den Wert $V_{ofs}$ reduziert dargestellt werden, damit korrekte Absolutwerte am Monitor ablesbar sind.

Bei einer Anordnung gemäß Figur 1, bei dem der Druckmesswandler 16 direkt mit dem Monitor verbunden ist, gilt, dass die Monitoreingangsspannung $V_{mon}$ gleich der Ausgangsspannung $V_{out}$ des Druckmesswandlers 16 ist, also:

$$V_{mon} = V_{out} \qquad (4).$$

Folglich erhält man

$$(V_{B}s)(p + p_{ofs}) = (V_{B}s)(p_{mon} + p_{mofs}) \qquad (5).$$

Dabei ist $p_{mon}$ der laufende Druckwert, der am Monitor angezeigt wird und $p_{mofs}$ der interne Offset-Kompensationswert. Durch Umstellung der Gleichung (5) erhält man Folgendes:

$$p_{mon} = p + p_{ofs} - p_{mofs} \qquad (6)$$

Damit am Monitor die korrekten Absolutwerte ausgelesen werden können, muss gelten:

$$p_{mon} = p \qquad (7)$$

oder

$$p_{mofs} = p_{ofs} \qquad (8)$$

Da jeder Druckmesswandler einen unterschiedlichen Wert $p_{ofs}$ aufweist, muss der Wert $p_{mofs}$ für jeden einzelnen Druckmesswandler am Monitor 18 derart eingestellt werden, dass letztendlich gilt $p_{mon}$ gleich p.

Für Figur 1 ergibt sich zur Durchführung eines Nullpunktabgleichs folgender Ablauf:

Zum Zeitpunkt $t_1$ wird das Ventil 14 auf eine Abgleichstellung umgestellt, bei der die Druckmessleitung 12 hin zum Patienten unterbrochen wird und die zwischen dem Ventil 14 und dem Druckmesswandler 16 stehende Flüssigkeitssäule dem Umgebungsluftdruck ausgesetzt wird. Der am Monitor 18 angezeigte Druck in mmHg sollte nun auf einer Nulllinie des Monitors liegen. Aufgrund der unterschiedlichen Offset-Spannungen $V_{ofs}$ von unterschiedlichen Druckmesswandlern 16 ist dies in aller Regel nicht der Fall. Folglich muss zu einem Zeitpunkt $t_2$ eine das System 10 bedienende Person 22 von der patientenseitigen Seite A hin zum Monitor B sich bewegen, und dort die Nulllinie des Monitors 18 händisch auf einen Wert Null einstellen. Dadurch wird gewährleistet, dass in der Nullpunktabgleichstellung des Ventils 14 am Monitor 18 tatsächlich null mmHg angezeigt werden. Zu einem Zeitpunkt $t_3$ muss dann die Person 22 wieder auf die Patientenseite A wechseln, um dort das Ventil 14 in die Betriebslage zu verstellen, so dass die flüssigkeitsgefüllte Druckmessleitung 12 mit dem Druckmesswandler 16 verbunden wird. Die am Druckmesswandler 16 anstehenden Drucksignale werden dann mit korrekten Absolutwerten am Monitor 18 angezeigt.

Bei dem beschriebenen, vorbekannten Verfahren zur Durchführung eines Nullpunktabgleichs ist es folglich erforderlich, dass die das System 10 bedienende Person 22 von der Patientenseite A zum Zeitpunkt $t_2$ zur Monitorseite B wechseln muss, und danach anschließend zum Zeitpunkt $T_3$ wieder zur Patientenseite A. Insgesamt ist das Verfahren vergleichsweise aufwändig und dauert vergleichsweise lang.

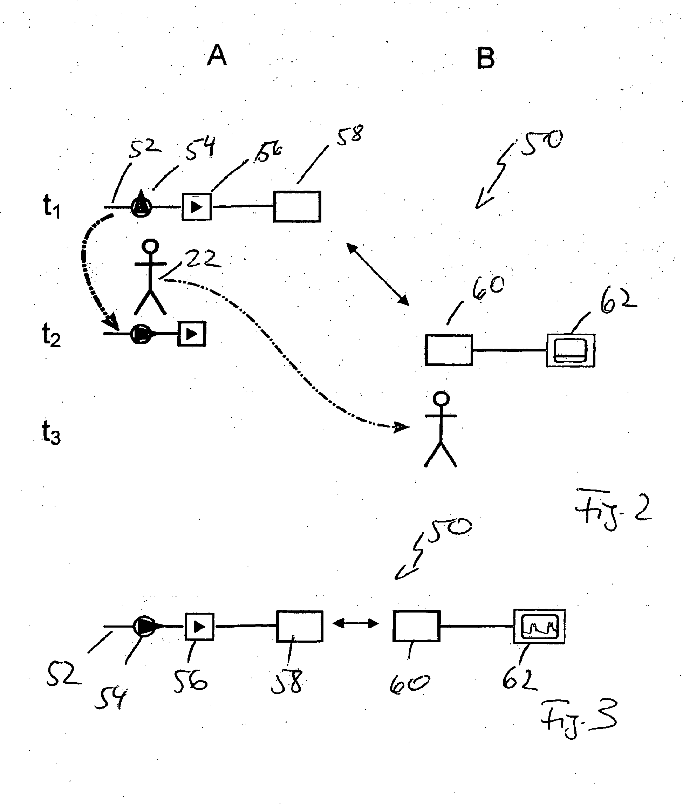

In der Figur 2 ist ein erfindungsgemäßes Durchführen des Nullpunktabgleichs bei einem erfindungsgemäßen Patientenüberwachungssystem 50 dargestellt.

Auf der Patientenseite A ist dabei, entsprechend dem System 10, eine Druckmessleitung 52 vorgesehen, mit welcher am Patienten abgenommene Drucksignale über ein Ventil 54 einem Druckmesswandler 56 zugeführt werden. Der Druckmesswandler 56, der die Drucksignale in elektrische Signale umwandelt, ist dabei mit einer Sendeeinheit 58 verbünden. Die Sendeeinheit 58 kommuniziert kabellos, insbesondere über Funk beziehungsweise Bluetooth, mit einer Empfangseinheit 60, welche mit einem Monitor 62 verbunden ist. Da beim Patientenüberwachungssystem 50, anders als beim Patientenüberwachungssystem 10 gemäß Figur 1, der Monitor 62 nicht unmittelbar mit dem Druckmesswandler 56 verbunden ist, gilt nicht als zwingende Voraussetzung, dass die Ausgangsspannung $V_{out}$ des Druckmesswandlers 56 gleich der Monitoreingangsspannung $V_{mon}$ ist, also

$$V_{mon} \neq V_{out} \qquad\qquad (8).$$

Gemäß der Erfindung ist vorgesehen, dass die von der Sendeeinheit ausgesendeten Messdaten bereits um die Offset-Spannung $V_{ofs}$ bereinigt sind; das heißt bei den von der Sendeeinheit 58 versendeten Messdaten handelt es sich um Messdaten mit korrekten Absolutwerten. Insofern gilt hier:

$$p_{ofs} = p_{mofs} = 0 \qquad\qquad (9)$$

und folglich auch

$$(V_B s)(p + 0) = (V_B s)(p_{mon} + 0) \qquad\qquad (10).$$

Da am Monitor keine Kompensation der Offset-Spannung $V_{ofs}$ durchzuführen ist, wie sie gemäß dem Stand der Technik nach Figur 1 zu erfolgen hat, gilt zudem

$$p_m = p \qquad\qquad (11).$$

Im Einzelnen wird der erfindungsgemäße Nullpunktabgleich gemäß Figur 2 wie folgt durchgeführt:

Zum Zeitpunkt $t_1$ wird das Ventil 54 auf die Abgleichstellung gestellt, das heißt die zwischen dem Ventil 54 und dem Druckmesswandler 56 anstehende Flüssigkeit wird dem Umgebungsdruck $p_0$ ausgesetzt. Die daraus resultierende, vom Druckmesswandler 56 erzeugte Offset-Spannung $V_{ofs}$ wird gemessen und in der Sendeeinheit 58 zwischengespeichert. Danach wird zum Zeitpunkt $t_2$ von der Bedienperson 22 das Ventil 54 in die Betriebsstellung umgelegt. Die Bedienperson 22 hat dadurch den Ort A nicht zu wechseln.

Im Betrieb sendet dann die Sendeeinheit 58 Messdaten an die Empfangseinheit 60, welche um den Wert der Offset-Spannung $V_{ofs}$ bereinigt sind, beziehungsweise bei welchen die Offset-Spannung $V_{ofs}$ abgezogen wurde. Insofern versendet die Sendeeinheit 58 Messdaten mit korrekten Absolutwerten.

Um sicherzustellen, dass am Monitor 62 auch die korrekten Absolutwerte angezeigt werden, ist es erforderlich, dass zu einem Zeitpunkt $t_3$, der allerdings auch vor dem Zeitpunkt $t_1$ liegen kann, auf der Seite B des Monitors 62 beziehungsweise des Empfängers 60, die Nulllinie des Monitors 62 einmalig eingestellt wird. Dazu wird von der Empfangseinheit 60 ein Basissignal, welches beispielsweise durch Drücken einer entsprechenden Taste an der Empfangseinheit generiert wird, an den Monitor 62 gesendet. Das Basissignal entspricht dabei insbesondere einem von der Sendeeinheit versendeten Signal, das aus dem Umgebungsluftdruck $p_0$ resultiert.

Während das Basissignal von der Empfangseinheit 60 zum Monitor 62 gesendet wird, kann beim Monitor 62 folglich die Nulllinie des Monitors auf den Wert Null mmHg eingestellt werden. Solange die Empfangseinheit 60 am Monitor 62 angeschlossen bleibt, muss dieser Abgleich nicht wiederholt werden. Insofern muss der Weg der Bedienperson von der Patientenseite A zur Monitorseite B lediglich einmal zurückgelegt werden.

Figur 3 zeigt das erfindungsgemäße Patientenüberwachungssystem dann im Betrieb nach Durchführung des Nullpunktabgleichs.

Beim Patientenüberwachungssystem 50 kann vorteilhafterweise vorgesehen sein, dass an der Sendeeinheit 58 eine Funktion beziehungsweise Taste vorgesehen ist, mit welcher ein Nullpunktabgleichsbefehl an die Empfangseinheit 60 versendet werden kann. Aufgrund des Nullpunktabgleichsbefehls generiert dann die Empfangseinheit 60 ein Nullpunktabgleichssignal, welches an den Monitor 62 übersendet wird. Der Monitor 62, der hierfür eine spezielle Schnittstelle vorsehen kann, kann dann aufgrund des Nullpunktabgleichssignals eine automatische Einstellung der Nulllinie auf den Wert Null mmHg vornehmen. Bei einem derartigen System erfolgt also der Nullpunktabgleich vollautomatisch von der Patientenseite A aus, lediglich durch Betätigen einer dort vorgesehenen Funktion beziehungsweise Taste.

Die Empfangseinheit 60 und der Monitor 62 können gemäß der Erfindung auch als eine Baugruppe ausgebildet sein, das heißt dass der Monitor 62 einerseits in der Empfangseinheit 60 integriert sein kann und/oder dass die Empfangseinheit 60 im Monitor 62 integriert sein kann.

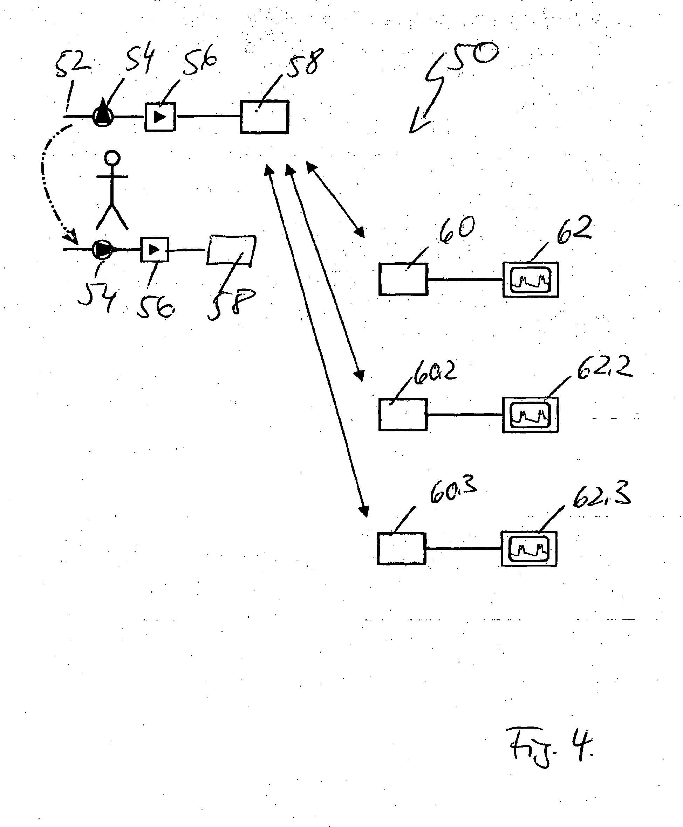

In der Figur 4 ist die erfindungsgemäße Überwachungseinrichtung 50 entsprechend Figur 2 dargestellt, wobei nicht nur eine Empfangseinheit 60 vorgesehen ist, sondern zusätzlich zwei weitere Empfangseinheiten 60.2 und 60.3 mit zugehörigen weiteren Monitoren 62.2 und 62.3.

Die Empfangseinheit 60 samt Monitor 62 kann beispielsweise in einem Vorbereitungsraum eines Operationssaals angeordnet sein. Die Empfangseinheit 60.2 samt Monitor 62.2 kann beispielsweise in einem Operationssaal angeordnet sein. Die Empfangseinheit 60.3 samt Monitor 62.3 kann insbesondere in einem Aufwachraum vorgesehen sein. Zur Durchführung des Nullpunktabgleichs kann auf der Patientenseite das Ventil 54, wie bereits beschrieben, in die Nullpunktabgleichsstellung verstellt werden und somit in der Sendeeinheit 58 die Offset-Spannung $V_{ofs}$ gemessen und gespeichert werden. Innerhalb einer zu wählbaren Zeit kann das Ventil 54 in die Betriebsstellung umgeschaltet werden. Danach können, falls dies nicht bereits erfolgt ist, Basissignale an den Empfangseinheiten 60, 60.2 und 60.3 erzeugt werden, zur einmaligen Nulllinieneinstellung an den Monitoren 62, 62.2 und 62.3.

Wie beschrieben, ist diese monitorseitige Einstellung nur einmalig durchzuführen, da die Berücksichtigung der Offset-Spannung $V_{ofs}$ nicht empfangseinheitsseitig, sondern sendeeinheitsseitig erfolgt.

**Patentansprüche**

**1.** Verfahren zur Durchführung eines Nullpunktabgleichs eines Patientenüberwachungssystems (50) mit einer am Patienten abgenommene Messdaten über eine Funkstrecke versendenden Sendeeinheit (58) und mit wenigstens einer Empfangseinheit (60) zum Empfang der von der Sendeeinheit (58) ausgesendeten Messdaten, wobei die am Patient abgenommenen Messdaten Drucksignale sind, die über eine flüssigkeitsgefüllte Druckmessleitung (52) einem die Drucksignale in elektrische Signale umwandelnden und an die Sendeeinheit (58) weiterleitenden Druckmesswandler (56) zugeführt werden, und wobei die von der Empfangseinheit (60) empfangenen Messdaten an einem Monitor (62) angezeigt werden, wobei zur Durchführung des Nullpunktabgleichs die flüssigkeitsgefüllte Druckmessleitung (52) hin zum Patienten unterbrochen und dem Umgebungsluftdruck ausgesetzt wird, und wobei die aus dem Umgebungsluftdruck $p_0$ resultierende, vom Druckmesswandler (56) erzeugte Offset-Spannung $V_{ofs}$ in der Sendeeinheit (58) bestimmt wird, **dadurch gekennzeichnet, dass** im Normalbetrieb die von der Sendeeinheit (58) versendeten Messdaten um den Wert der Offset-Spannung $V_{ofs}$ bereinigt an die Empfangseinheit (60) versendet werden.

**2.** Verfahren nach Anspruch 1, **dadurch gekennzeichnet, dass** mit der Empfangseinheit (60) ein Basissignal zum Abgleichen der am Monitor (62) angezeigten Signale generierbar ist, aufgrund dessen die Nulllinie des Monitors (62) auf einen Basiswert einstellbar ist.

**3.** Verfahren nach Anspruch 1 oder 2, **dadurch gekennzeichnet, dass** das Basissignal einem von der Sendeeinheit (58) versendeten Signal, das aus dem Umgebungsluftdruck $p_0$ resultiert, entspricht und dass der Basiswert einem Nullwert entspricht.

**4.** Verfahren nach Anspruch 1, 2 oder 3, **dadurch gekennzeichnet, dass** die Nulllinie des Monitors (62) händisch einstellbar ist.

**5.** Verfahren nach Anspruch 1, 2 oder 3, **dadurch gekennzeichnet, dass** die Nulllinie des Monitors (62) aufgrund eines von der Empfangseinheit (60) generierten Nullpunktabgleichssignals automatisch eingestellt wird.

**6.** Verfahren nach Anspruch 5, **dadurch gekennzeichnet, dass** während oder nach der Speicherung der Offset-Spannung $V_{ofs}$ in der Sendeeinheit (58) ein Nullpunktabgleichsbefehl an die Empfangseinheit (60) versendet wird, aufgrund dessen das Nullpunktabgleichssignal von der Empfangseinheit (60) generiert und die Nulllinie des Monitors (62) entsprechend händisch einstellbar ist und/oder automatisch eingestellt wird.

**7.** Patientenüberwachungssystem (50) mit einer am Patienten abgenommene Messdaten über eine Funkstrecke versendenden Sendeeinheit (58) und mit wenigstens einer Empfangseinheit (60) zum Empfangen der von der Sendeeinheit (58) ausgesendeten Messdaten, geeignet und/oder vorgesehen zur Durchführung des Verfahrens nach wenigstens einem der vorhergehenden Ansprüche, wobei in oder an der Druckmessleitung (52) ein Ventil (54) vorgesehen ist, welches in einer Betriebsstellung eine Verbindung zwischen dem Patienten und dem Druckmesswandler (56) herstellt und welches zur Durchführung eines Nullpunktabgleichs in einer Abgleichsstellung eine Verbindung zwischen einer am Umgebungsluftdruck anstehenden Flüssigkeit und dem Druckmesswandler (56) herstellt, und wobei die Sendeeinheit (58) Auswertmittel umfasst, welche die in der Abgleichsstellung des Ventils (54) aus dem Umgebungsluftdruck $p_0$ resultierende, vom Druckmesswandler (56) erzeugte Offset-Spannung $V_{ofs}$ bestimmen, **dadurch gekennzeichnet, dass** die Auswertmittel die von der Sendeeinheit (58) an die Empfangseinheit (60) versendeten Messdaten um den Wert der Offset-Spannung $V_{ofs}$ bereinigen.

**8.** System (50) nach Anspruch 7, **dadurch gekennzeichnet, dass** die Sendeeinheit (58) eine Speicherfunktion aufweist, die zur Speicherung der Offset-Spannung $V_{ofs}$ betätigbar ist.

**9.** System (50) nach Anspruch 7 oder 8, **dadurch gekennzeichnet, dass** die Empfangseinheit (60) eine Abgleichfunktion aufweist, die zur Generierung eines Basissignals zum Abgleichen der am Monitor (62) angezeigten Signale, betätigbar ist, wobei aufgrund des Basissignals die Nulllinie des Monitors (62) auf einen Basiswert einstellbar ist.

**10.** System (50) nach Anspruch 7, 8 oder 9, **dadurch gekennzeichnet, dass** die Sendeeinheit (58) eine Abgleichfunktion und die Empfangseinheit (60) Mittel zur Generierung eines Nullpunktabgleichsbefehls derart aufweist, dass durch Betätigung der Abgleichfunktion von der Sendeeinheit ein Nullpunktabgleichsbefehls an die Empfangseinheit (60) übersendet wird und aufgrund des Nullpunktabgleichsbefehls in der Empfangseinheit (60) ein Nullpunktabgleichssignal generiert wird.

**11.** System (50) nach Anspruch 10, **dadurch gekennzeichnet, dass** die Empfangseinheit (60) Mittel zur automatischen Einstellung der Nulllage des Monitors derart aufweist, dass die Nulllinie des Monitors aufgrund des Nullpunktabgleichssignals automatisch eingestellt wird.

**12.** System (50) nach Anspruch 7, 8 oder 9, **dadurch gekennzeichnet, dass** der Monitor in die Empfangseinheit integriert ist.

**13.** System (50) nach wenigstens einem der Ansprüche 7 bis 10, **dadurch gekennzeichnet, dass** der Monitor (62) separat von der Empfangseinheit (60) ausgebildet ist und einen Anschluss aufweist, über den das von der Empfangseinheit (60) generierte Nullpunktabgleichssignals dem Monitor zuführbar ist.

**Claims**

1. Method for carrying out a zero point comparison in a patient monitoring system (50) with a transmitter unit (58) transmitting via a radio link measurement data taken from the patient, and with at least one receiver unit (60) for receiving the measurement data transmitted by the transmitter unit (58), wherein the measurement data taken from the patient are pressure signals which are supplied via a fluid-filled pressure measuring line (52) to a pressure transducer (56) which converts the pressure signals into electrical signals and passes these on to the transmitter unit (58), and wherein the measurement data received by the receiver unit (60) are displayed on a monitor (62), wherein for performance of the zero point comparison, the fluid-filled pressure measuring line (52) to the patient is interrupted and exposed to ambient air pressure, and wherein the offset voltage $V_{ofs}$ generated by the pressure transducer (56) and resulting from the ambient air pressure $p_0$ is determined in the transmitter unit (58), **characterized in that** in normal operation, the measurement data transmitted by the transmitter unit (58) are transmitted to the receiver unit (60) cleaned by the value of the offset voltage $V_{ofs}$.

2. Method according to claim 1, **characterized in that** using the receiver unit (60), a base signal can be generated for comparison of the signals displayed on the monitor (62), and on the basis of which the zero line of the monitor (62) can be set to a base value.

3. Method according to claim 1 or 2, **characterized in that** the base signal corresponds to a signal transmitted by the transmitter unit (58) which results from the ambient air pressure $p_0$, and that the base value corresponds to a zero value.

4. Method according to claim 1, 2 or 3, **characterized in that** the zero line of the monitor (62) can be adjusted by hand.

5. Method according to claim 1, 2 or 3, **characterized in that** the zero line of the monitor (62) is adjusted automatically on the basis of a zero point comparison signal generated by the receiver unit (60).

6. Method according to claim 5, **characterized in that** during or after storage of the offset voltage $V_{ofs}$ in the transmitter unit (58), a zero point comparison command is sent to the receiver unit (60), on the basis of which the zero point comparison signal is generated by the receiver unit (60) and the zero line of the monitor (62) can be adjusted by hand and/or is adjusted automatically accordingly.

7. Patient monitoring system (50) with a transmitter unit (58) transmitting via a radio link measurement data taken from the patient, and with at least one receiver unit (60) for receiving the measurement data transmitted by the transmitter unit (58), suitable and/or configured for performance of the method according to at least one of the preceding claims, wherein a valve (54) is provided in or on the pressure measuring line (52) which in an operating position creates a connection between the patient and the pressure transducer (56) and, to perform a zero point comparison, in a comparison position creates a connection between a fluid under ambient air pressure and the pressure transducer (56), and wherein the transmitter unit (58) comprises analysis means which determine the offset voltage $V_{ofs}$ generated by the pressure transducer (56) and resulting from the ambient air pressure $p_0$ when the valve (54) is in the comparison position, **characterized in that** the analysis means clean the measurement data transmitted by the transmitter unit (58) to the receiver unit (60) by the value of the offset voltage $V_{ofs}$.

8. System (50) according to claim 7, **characterized in that** the transmitter unit (58) has a memory function which can be actuated to store the offset voltage $V_{ofs}$.

9. System (50) according to claim 7 or 8, **characterized in that** the receiver unit (60) has a comparison function which can be actuated to generate a base signal for comparison with the signals displayed on the monitor (62), wherein on the basis of the base signal, the zero line of the monitor (62) can be set to a base value.

10. System (50) according to claim 7, 8 or 9, **characterized in that** the transmitter unit (58) has a comparison function and the receiver unit (60) has means for generating a zero point comparison command such that by activation of the comparison function of the transmitter unit, a zero point comparison command is transmitted to the receiver unit (60) and on the basis of the zero point comparison command, a zero point comparison signal is generated in the receiver unit (60).

11. System according to claim 10, **characterized in that** the receiver unit (60) has means for automatic adjustment of the zero point of the monitor such that the zero line of the monitor is adjusted automatically on the basis of the zero point comparison signal.

**12.** System (50) according to claim 7, 8 or 9, **characterized in that** the monitor is integrated in the receiver unit.

**13.** System (50) according to any of claims 7 to 10, **characterized in that** the monitor (62) is formed separately from the receiver unit (60) and has a connection via which the zero point comparison signal generated by the receiver unit (60) can be supplied to the monitor.

**Revendications**

**1.** Procédé de réglage au point zéro d'un système de surveillance de patients (50) comportant une unité émettrice (58), qui envoie par liaison radio des données de mesures prises sur un patient, et au moins une unité réceptrice (60) destinée à recevoir les données de mesures envoyées par l'unité émettrice (60), les données de mesures prises sur ledit patient étant des signaux de pression, qui sont envoyés par l'intermédiaire d'une conduite de mesure de pression (52) remplie de liquide à un convertisseur de mesure de pression (56) qui convertit les signaux de pression en signaux électriques et les transmet à l'unité émettrice (58), et les données de mesures reçues par l'unité réceptrice (60) étant affichées sur un écran (62), la conduite de mesure de pression (52) remplie de liquide, pour le réglage au point zéro, étant obturée en direction du patient et soumise à la pression de l'air ambiant, la tension d'offset Vos générée par le convertisseur de mesure de pression (56) et résultant de la pression de l'air ambiant $p_0$ étant déterminée dans l'unité émettrice (58), **caractérisé en ce que**, en fonctionnement normal, les données de mesures envoyées par l'unité émettrice (58) sont corrigées de la valeur de la tension d'offset $V_{ofs}$ avant d'être envoyées à l'unité réceptrice (60).

**2.** Procédé selon la revendication 1, **caractérisé en ce qu'**un signal de base, qui sert à corriger les signaux affichés sur l'écran (62), et sur la base duquel la ligne zéro de l'écran (62) peut être réglée sur une valeur de base, peut être généré par l'unité réceptrice (60).

**3.** Procédé selon la revendication 1 ou 2, **caractérisé en ce que** le signal de base correspond à un signal émis par l'unité émettrice (58), qui résulte de la pression de l'air ambiant $p_0$, et **en ce que** la valeur de base correspond à une valeur nulle.

**4.** Procédé selon la revendication 1, 2 ou 3, **caractérisé en ce que** la ligne zéro de l'écran (62) est réglable manuellement.

**5.** Procédé selon la revendication 1, 2 ou 3, **caractérisé en ce que** la ligne zéro de l'écran (62) est réglée automatiquement sur la base d'un signal de réglage au point zéro généré par l'unité réceptrice (60).

**6.** Procédé selon la revendication 5, **caractérisé en ce que** pendant ou après la mise en mémoire de la tension d'offset $V_{ofs}$ dans l'unité émettrice (58), un ordre de réglage au point zéro est envoyé à l'unité réceptrice (60), sur la base duquel le signal de réglage au point zéro est généré par l'unité réceptrice (60), et la ligne zéro de l'écran (62) peut être réglée manuellement et/ou est réglée automatiquement de manière correspondante.

**7.** Système de surveillance de patients (50) comportant une unité émettrice (58), qui envoie par liaison radio des données de mesures prises sur un patient, et au moins une unité réceptrice (60) destinée à recevoir les données de mesures envoyées par l'unité émettrice (60), adapté et/ou conçu pour mettre en oeuvre le procédé selon au moins l'une des revendications précédentes, une soupape (54), laquelle, dans une position de fonctionnement, établit une liaison entre le patient et le convertisseur de mesure de pression (56), et laquelle, pour le réglage au point zéro, dans une position de réglage, établit une liaison entre un liquide présent sur la pression de l'air ambiant et le convertisseur de mesure de pression (56), étant présente dans ou sur la conduite de mesure de pression (52), et l'unité émettrice (58) comprenant des moyens d'évaluation, lesquels déterminent la tension d'offset Vos générée par le convertisseur de mesure de pression (56) et résultant de la pression de l'air ambiant $p_0$, **caractérisé en ce que** les valeurs d'évaluation corrigent les données de mesure envoyées par l'unité émettrice (58) à l'unité réceptrice (60) de la valeur de la tension d'offset $V_{ofs}$.

**8.** Système (50) selon la revendication 7, **caractérisé en ce que** l'unité émettrice (58) comprend une fonction de mise en mémoire qui peut être actionnée pour la mise en mémoire de la tension d'offset $V_{ofs}$.

**9.** Système (50) selon la revendication 7 ou 8, **caractérisé en ce que** l'unité réceptrice (60) comprend une fonction de réglage qui peut être actionnée pour générer un signal de base servant à régler les signaux affichés sur l'écran (62), la ligne zéro de l'écran (62) pouvant être réglée sur une valeur de base en se basant sur le signal de base.

**10.** Système (50) selon la revendication 7, 8 ou 9, **caractérisé en ce que** l'unité émettrice (58) comprend une fonction de réglage et l'unité réceptrice (60) comprend des moyens servant à générer un ordre de réglage au point zéro, de telle manière que par l'actionnement de la fonction de réglage par l'unité émettrice, un ordre de réglage au point zéro est envoyé à l'unité réceptrice (60) et un signal de réglage au point zéro est généré dans l'unité réceptrice (60) sur la base de l'ordre de réglage au point zéro.

**11.** Système (50) selon la revendication 10, **caractérisé en ce que** l'unité réceptrice (60) comprend des moyens servant au réglage automatique de la position zéro de l'écran, de telle manière que la ligne zéro de l'écran est réglée automatiquement sur la base du signal de réglage au point zéro.

**12.** Système (50) selon la revendication 7, 8 ou 9, **caractérisé en ce que** l'écran est intégré dans l'unité réceptrice.

**13.** Système (50) selon au moins l'une des revendications 7 à 10, **caractérisé en ce que** l'écran (62) est formé séparément de l'unité réceptrice (60) et comprend une borne par l'intermédiaire de laquelle le signal de réglage au point zéro généré par l'unité réceptrice (60) peut être amené à l'écran.

A          B

t₁

t₂

t₃

Fig. 1

A          B

Fig. 2

Fig. 3

Fig. 4.

Fig. 5

Fig. 6

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1574164 A1

- US 5566676 A